# EUROPEAN PATENT SPECIFICATION

(11) **EP 3 734 274 B1**
(45) Date of publication and mention of the grant of the patent: **23.04.2025**
(21) Application number: 20175351.4
(22) Date of filing: 23.12.2008
(51) Int. Cl.: G01N 33/49

(54) **SYSTEM FOR MEASURING VISCOELASTIC PROPERTIES OF BLOOD**
SYSTEM ZUR MESSUNG VISKOELASTISCHER BLUTEIGENSCHAFTEN
SYSTÈME D'ÉVALUATION DES PROPRIETES VISCOELASTIQUES DU SANG

(43) Date of publication of application: 04.11.2020
(62) Divisional of application: 13167983.9
(73) Proprietor: C A Casyso GmbH, 4052 Basel (CH)
(72) Inventor: Romero-Galeano, José Javier, 85570 Markt Schwaben (DE); Schubert, Axel, 07743 Jena (DE); Kessler, Max, 81539 München (DE)
(74) Representative: Müller-Boré & Partner Patentanwälte PartG mbB

(56) References cited:
- WO-A1-2005/106467
- US-A- 4 443 408
- US-A1- 2002 081 741
- US-A1- 2004 131 500
- US-A1- 2008 297 169

## Description

### FIELD OF THE INVENTION

The present invention relates to a system for evaluation of hemostasis.

### BACKGROUND

It is essential for survival that a wound stops bleeding, i.e. that the body possesses an adequate mechanism for haemostasis. The process of blood clotting can be activated in the case of injuries or inflammations by either extrinsic or intrinsic factors, e.g. tissue factor (TF) or Hagemann factor (F XII), respectively. Both activation channels are continued in a common branch of the cascade resulting in thrombin formation. The thrombin itself finally initiates the formation of fibrin fibres which represent the protein backbone of blood clots.

The other main constituent of the final blood clot are the thrombocytes which are interconnected by the fibrin fibres and undergo a number of physiological changes during the process of coagulation. Within limits a lack of thrombocytes can be substituted by an increased amount of fibrin or vice versa. This is reflected in the observation that the thrombocyte counts as well as the fibrinogen concentration varies even within a healthy population.

Various methods have been introduced to assess the potential of blood to form an adequate clot and to determine the blood clots stability. Common laboratory tests such as thrombocyte counts or the determination of fibrin concentration provide information on whether the tested component is available in sufficient amount but lack in answering the question whether the tested component works properly under physiological conditions (e.g. the polymerisation activity of fibrinogen under physiological conditions can not be assessed by common optical methods). Besides that, most laboratory tests work on blood-plasma and therefore require an additional step for preparation and additional time which is unfavourable especially under POC (point of care) conditions.

Another group of tests which overcomes these problems is summarized by the term "viscoelastic methods". The common feature of these methods is that the blood clot firmness (or other parameters dependent thereon) is continuously determined, from the formation of the first fibrin fibres until the dissolution of the blood clot by fibrinolysis. Blood clot firmness is a functional parameter, which is important for haemostasis in vivo, as a clot must resist blood pressure and shear stress at the site of vascular injury. Clot firmness results from multiple interlinked processes: coagulation activation, thrombin formation, fibrin formation and polymerization, platelet activation and fibrin-platelet interaction and can be compromised by fibrinolysis. Thus, by the use of viscoelastic monitoring all these mechanisms of the coagulation system can be assessed.

A common feature of all these methods used for coagulation diagnosis is that the blood clot is placed in the space between a cylindrical pin and an axially symmetric cup and the ability of the blood clot to couple those two bodies is determined.

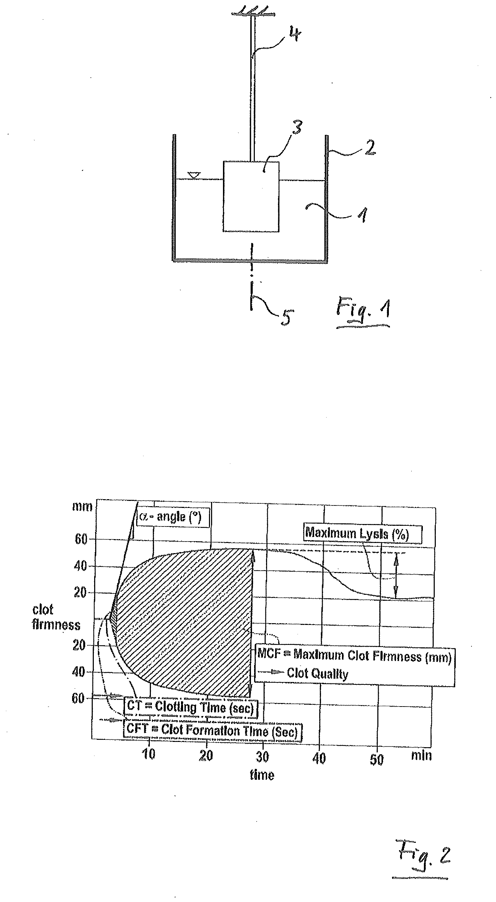

The first viscoelastometric method was called "thrombelastography" (Hartert H: Blutgerinnungsstudien mit der Thrombelastographie, einem neuen Untersuchungsverfahren. Klin Wochenschrift 26:577-583, 1948). As illustrated in FIG. 1, in the thromboelastography, the sample as a sample liquid 1 is placed in a cup 2 that is periodically rotated to the left and to the right by about 5°, respectively. A probe pin 3 is freely suspended by a torsion wire 4. When a clot is formed it starts to transfer the movement of the cup 2 to the probe pin 3 against the reverse momentum of the torsion wire 4. The movement of the probe pin 3 as a measure for the clot firmness is continuously recorded and plotted against time. For historical reasons the firmness is measured in millimetres.

The result of a typical measurement of this kind is illustrated in FIG. 2. One of the most important parameters is the time between the activator induced start of the coagulation cascade and the time until the first long fibrin fibres have been build up which is indicated by the firmness signal exceeding a defined value. This parameter will be called clotting time or just CT in the following. Another important parameter is the clot formation time (CFT) which gives a measure for the velocity of the development of a clot. The CFT is defined as the time it takes for the clot firmness to increase from 2 to 20 mm. The maximum firmness a clot reaches during a measurement, further on referred to as maximum clot firmness or just MCF, is also of great diagnostic importance.

Modifications of the original thromboelastography technique (Hartert et al. (US 3,714,815) have been described by Cavallari et al. (US 4,193,293), by Do et al. (US 4,148,216), by Cohen (US 6,537.819). A further modification by Calatzis et al. (US 5,777,215) illustrated in FIG. 3 is known under the term thromboelastometry.

Contrary to the modifications mentioned above, thromboelastometry is based on a cup 2 fixed in a cup holder 12 while the probe pin 3 is actively rotated. For this purpose the probe pin 3 is attached to a shaft 6 which is suspended by a ball bearing 7 in a base plate 11 and has a spring 9 connected to it. An oscillating motion perpendicular to the drawing plane induced at the opposite end of the spring is transformed into a periodically rotation of the shaft 6 and the connected cup 2 around a rotation axis 5 by about 5° in each direction. As the sample liquid 1 begins to coagulate the motion amplitude of the shaft 6 which is detected by the deflection of a light beam from detecting means 10 and a mirror 9 starts to decrease.

During coagulation the fibrin backbone creates a mechanical elastic linkage between the surfaces of the blood-containing cup 2 and a probe pin 3 plunged therein. A proceeding coagulation process induced by adding one or more activating factor(s) can thus be observed. In this way, various deficiencies of a patient's haemostatic status can be revealed and can be interpreted for proper medical intervention.

A general advantage of viscoelastometric, e.g. thromboelastometric, techniques compared to other laboratory methods in this field therefore is that the coagulation process and the change of mechanical properties of the sample are monitored as a whole. This means that - in contrary to other laboratory methods mentioned above - thromboelastometry does not only indicate if all components of the coagulation pathways are available in sufficient amounts but also if each component works properly.

To obtain detailed information on the correct amount and function of the thrombocytes as well as the fibrinogen and certain factors nowadays there is an increasing amount of compounds available which activate or inhibit certain components of the coagulation system. This allows determining at which point of the coagulation system a problem is located.

For practical reasons theses compounds are usually injected into the disposable plastic cup which later on is used for the measurement by using a pipette (either a manual or an automatic one). In the last preparation step, after the blood or plasma sample has been added, the whole amount of sample (blood/plasma and the additional chemicals) is mixed by drawing it into the pipette tip and dispensing it into the cup again.

The possibility to activate or to inhibit certain components of the coagulation system is especially useful in conjunction with state-of-the-art thromboelastometers such as the ROTEM (Pentapharm GmbH, Munich, Germany) which allows conducting four measurements in parallel. This allows detailed information on the current status of the coagulation-situation of a patient to be achieved and therefore allows an appropriate therapy within several minutes.

This is of particular importance in case of patients struck by massive blood loss as it often occurs in context with multiple traumata or major surgery. The blood of such patients often is diluted due to infusions which are administered to replace the loss in volume. This leads to a decrease of the concentration of thrombocytes as well as coagulation factors including fibrinogen.

Main advantages of thromboelastometry and thromboelastography are the possibility to perform several differential tests in parallel in order to precisely determine which kinds of blood products are the appropriate medication, the possibility to perform the measurement at or close to the point of care (POC) and - compared to other methods - the relatively small amount of time until valid results are available.

On the other hand the operator has to perform a significant number of steps in order to start the measurement (preparation of the reagents, attachment of the probe pin and the cup to the instrument, pipeting and mixing the blood sample and the reagents, adjustment of computer settings, etc.) on which the time spent is considerable, especially in the case of surgery being performed.

Furthermore this rather complex preparation also increases the risk of operating errors. There have been several approaches to simplify the usage of thromboelastometers. The Rotem-System (Pentapharm GmbH, Munich, Germany) e.g. is supplied with an automatic pipette which simplifies the handling to a large degree and thereby decreases the risk of operating errors.

WO 2008093216 describes the approach to provide the adequate amount of each of the reagents needed for one specific test in a ready-to-use mixture. In order to prevent the reaction of the reagents prior to the measurement, they are supplied in a lyophilisate state. This is additionally advantageous as the reagents can be stored at room temperature. Using this approach the preparation is reduced to the steps of adding the blood sample into the reagent container, mixing of blood with the reagent and transferring the mixture to the instrument.

US 2007/0059840 A1 describes a hemostasis analysis device and method. The device includes a container for holding a sample to be tested and a bobber configured to be buoyantly suspended on the sample. A magnet is secured to the bobber. The container can be driven in an oscillating motion. An external magnetic field is generated adjacent to the bobber. A magnetic field strength detector detects changes in the magnetic field as a result of movement of the bobber and magnet responsive to the oscillating motion of the container and clotting of the sample.

Such a new measuring system entails acceptability problems and uncertainties for a user. Moreover, that analysis device does not fit in existing measuring systems. Therefore new systems have to be completely designed.

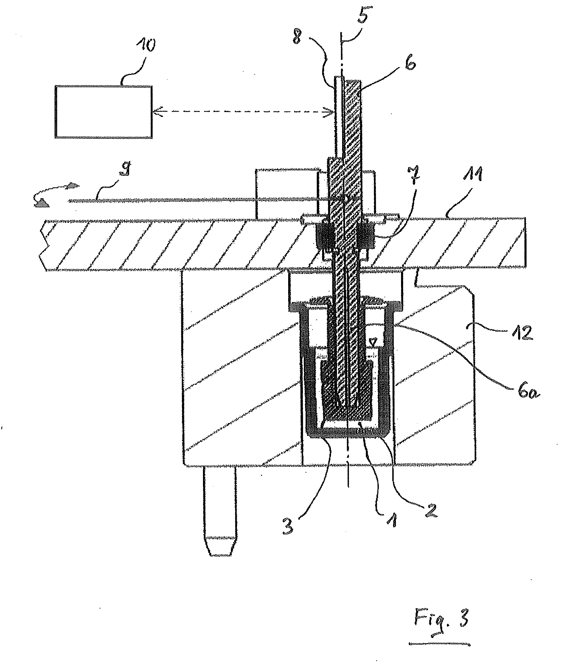

All these modifications lead to a significant improvement of handling of modern thromboelastometers and thromboelastographs, however, no successful approach to develop a widely automated technique has been made since Hartert's invention 60 years ago. One of the two main reasons of that is the fact that the measurement requires two disposable parts (cup and pin) being moved in relation to each other and thus have to be reversibly attached to different parts of the measurement device. E.g. in FIG. 3, the probe pin 3 is attached to the shaft 6 and the cup 2 to the cup holder 12, respectively. The other main reason is that different tests are required to get comprehensive information of a current bleeding status of a patient. These different tests require different reagents which have to be mixed with the blood sample.

WO 2005/106467 A1 describes a system and a method for determining a coagulation time, e.g., TT, PT, aPTT, and ACT, of a blood test sample deposited in a test cartridge. A cartridge housing having upper and lower major sides and a minor sidewall encloses a test chamber having a test chamber pivot element and is provided with a cartridge port for introducing a test sample into the test chamber. Ferromagnetic agitator vane leaflets extend from an agitator pivot element supported by the test chamber pivot element intermediate the upper and lower major sides for rotational motion. The agitator vane leaflets can be swept, in response to an external magnetic field, through the test sample in the absence of coagulation. A timer is started when the agitator movement is commenced whereupon the agitator moves freely. Resistance to agitator movement due to coagulation is detected, and the coagulation time is measured.

### SUMMARY OF THE INVENTION

It is a problem underlying the presented invention to provide a cartridge device for a measuring system for measuring viscoelastic characteristics of a sample liquid, in particular a blood sample.

Directly connected to this invention is the problem to provide a corresponding measuring system for measuring viscoelastic characteristics of a sample liquid, in particular the coagulation characteristics of a blood sample liquid.

It is a further problem underlying the invention to provide a method for measuring viscoelastic characteristics of a sample liquid using said measuring system. These problems are solved by the subject-matter of independent claim 1. Preferred embodiments are set forth in the dependent claims.

In a preferred embodiment the probe element comprises a probe pin to cooperate with the sample liquid and a connector section for a connection to the measuring system. The connector section is formed e.g. as a bore extending within the probe element and comprises frictional connection means which can be e.g. clip means or a thread. An insertion guide facilitates an insertion of a part, in particular a shaft, of a measuring system. Thereby the shaft can be connected securely to the probe element.

The measurement cavities can comprise bearing or supporting means for the probe element to align or hold the probe element prior to insertion of the shaft.

After the shaft has been inserted into the connector section, the shaft can be lifted to position the probe element at a working position.

In an alternative preferred embodiment the probe element is formed as a detachably fixed component part of the cover. An operator only has to attach the cartridge device to the measuring system the shaft being inserted into the probe element will detach the probe element from the cover and hold it securely in a position ready to carry out a measurement. Therefore the probe element comprises a fixing section for detachably fixing the probe element at fixing means of the cover.

After a measurement the cartridge device can be detached from the measuring system wherein the shaft is removed from the probe element. Then the probe element will seal the measurement cavity against the cover by means of e.g. a flange adapted to form a sealing. The cover retains the probe element within the measurement cavity.

It is preferred that the fixing means of the cover comprises clip means cooperating with corresponding clip means of the fixing section of the probe element.

In an alternative embodiment the fixing section of the probe element is integrally formed with the cover, the fixing means of the cover comprising a perforation.

The cover can be fixed on the cartridge body either by bonding or welding. In an alternative embodiment the cover is integrally formed with the cartridge body, e.g. made of a plastic material. It is also possible that the cover is made of a material which is different from the cartridge body. That can be done for example by two- or more-component-moulding.

The cartridge device further comprises receiving cavities formed therein for receiving the blood sample; reagent cavities for holding reagents; a ductwork connecting said cavities and the measurement cavities; and at least one pump means connected to the ductwork for transporting the sample liquid from the receiving cavities to the measurement cavities by means of the ductwork, wherein the cover covers and at least partially forms said cavities and said ductwork and forms at least partially the pump means.

In a further embodiment the reagent cavities are integrally formed with the pump means or/and with the measurement cavities or/and with one or more of the ductworks. The reagent cavity can be formed as a deep cavity or just a small place where reagent can be deposited. Thus the sample liquid being pumped through the ductwork and the pump means into the measurement cavity can be mixed with the reagent.

The pump means comprise at least one valve for a directed flow of the sample liquid in order to direct the pumped liquid into the measurement cavity.

The reagent or an additional reagent are stored in reagent receptacles which can be opened by external means.

In a further embodiment the reagent receptacles storing a reagent are integrated in the cover.

In another embodiment the reagent receptacles comprise a bottom part which can be opened by external means to discharge the reagent into the ductwork and/or into one of the cavities. The receptacle can be adapted as a blister receptacle, for example.

The reagents can be stored within the cartridge device in pulverized, solid or liquid form.

The cartridge device is further provided with reagents stored therein.

Filling in sample liquid, namely blood sample, can be done directly into the measurement cavity if no receiving cavity is provided. To this end the sample liquid can be injected through the cover via an opening or passage hole in the interface element or through a ductwork by an operator or by a control apparatus.

In case of a receiving cavity the sample liquid, namely blood sample, can be filled into the receiving cavity and be pumped by the pump means to the measuring cavity.

To fill in sample liquid, operate the pump means, add reagents and/or open the reagent receptacle the measuring system is equipped with a control apparatus. The control apparatus has means to access the pump means through a pump access formed as a passage of the interface element. Further the control apparatus can inject sample liquid through an inlet opening in the interface element into the receiving cavity. The control apparatus comprises also operating means to inject or to add reagents into the cartridge device as well as to open reagent receptacles.

### BRIEF DESCRIPTION OF THE DRAWINGS

Further features and advantages of the present invention will be evident from a description of embodiments with reference to the figures.

The figures are showing the following:
FIG. 1 is a schematic drawing of the principle of thromboelastography according to Hartert.
FIG. 2 is an exemplary diagram showing a typical thromboelastometric measurement.
FIG. 3 is a schematic drawing of the thromboelastometry.
FIG. 4 is a schematic drawing of a first cartridge device.
FIG. 5 is a schematic drawing of a variation of the first cartridge device.
FIG. 6 is a schematic drawing of another variation of the first cartridge device.
FIG. 7a is a schematic drawing of a first embodiment of a probe element.
FIG. 7b is a schematic drawing of the first embodiment of the probe element of

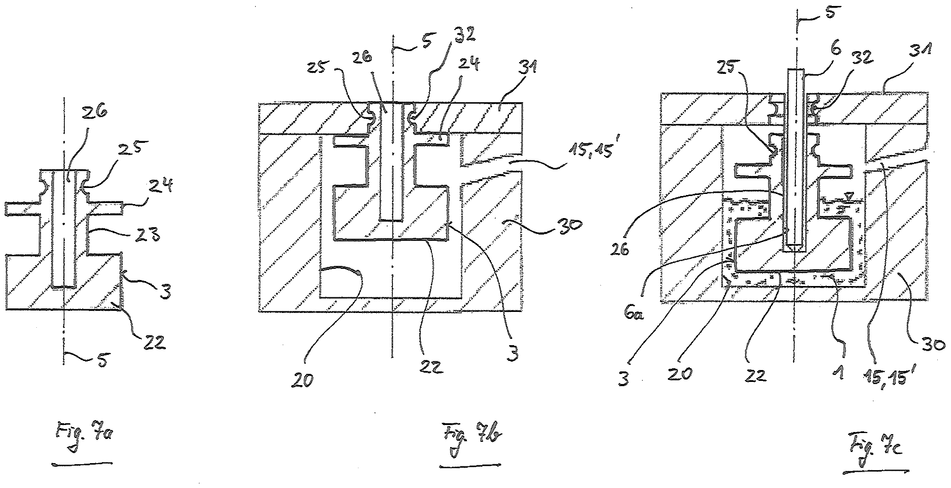

FIG. 7a within a measuring cavity of the first or a second cartridge device before use.
FIG. 7c is a schematic drawing of the first embodiment of the probe element of
FIG. 7a within a measuring cavity of the first or the second cartridge device in use.
Fig .8a...c are technical drawings of the preferred probe element of FIG. 7a.

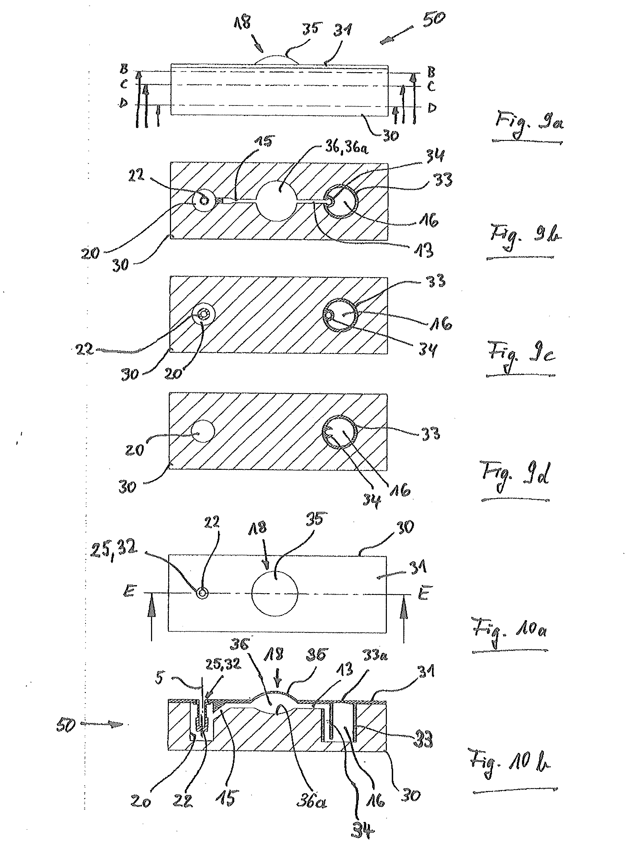

FIG. 9a is a side view of a third cartridge device.
FIG. 9b is a sectional view B-B of the cartridge device of FIG. 9a.
FIG. 9c is a sectional view C-C of the cartridge device of FIG. 9a.
FIG. 9d is a sectional view D-D of the cartridge device of FIG. 9a.
FIG. 10a is a top view of the cartridge device of FIG. 9a.
FIG. 10b is a sectional view E-E of the cartridge device of FIG. 10a.

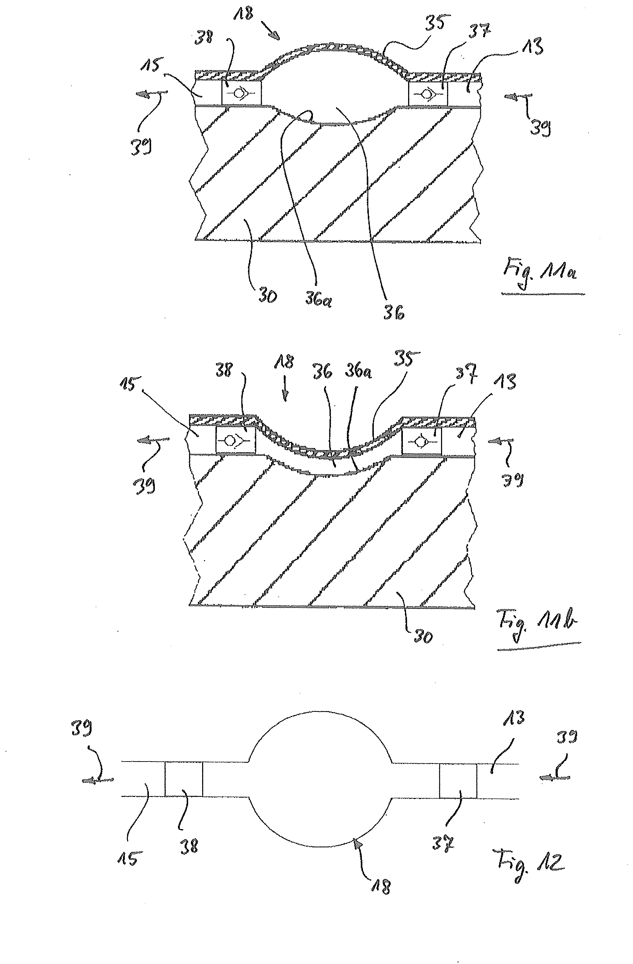

FIG. 11a is a sectional view of a pump means of the cartridge device of FIG. 9a.
FIG. 11b is a sectional view of the pump means of FIG. 11a in operated position.
FIG. 12 is a schematic top view of the pump means of FIG. 11a.

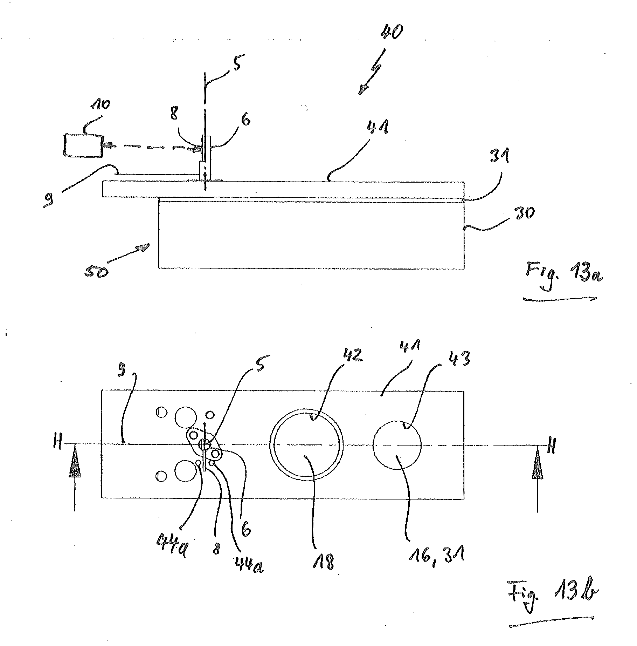

FIG. 13a is a side view of an embodiment of a measuring system according to the invention.
FIG. 13b is a top view of the measuring system of FIG. 13a.

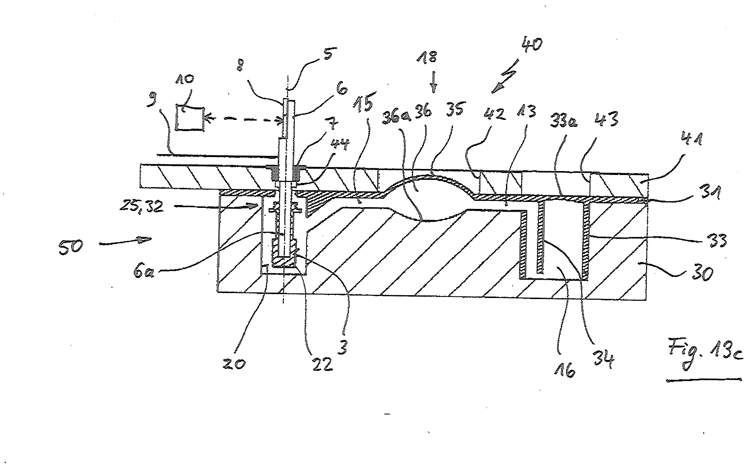

FIG. 13c is a sectional view H-H of the measuring system of FIG. 13b.

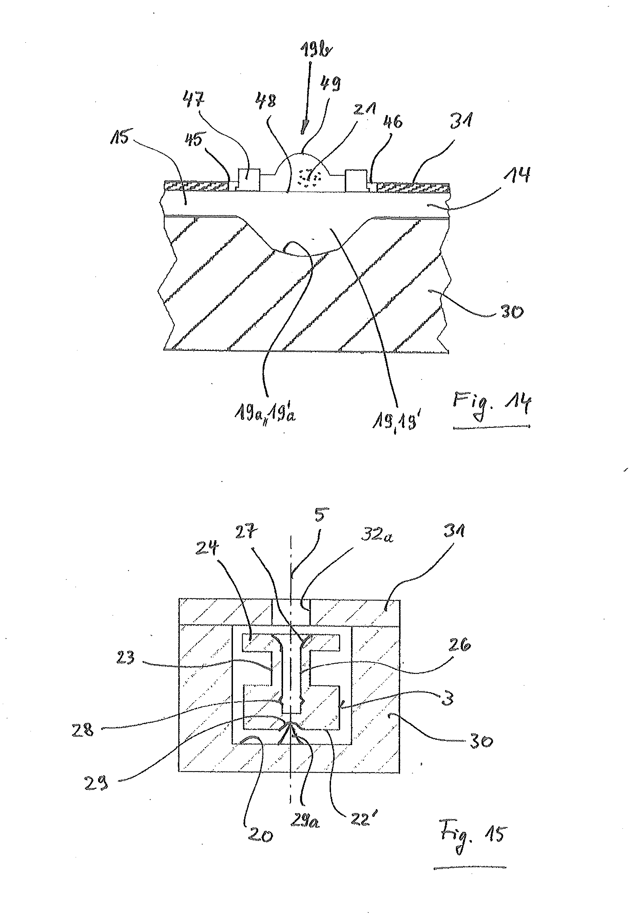

FIG. 14 is a sectional view of a reagent receptacle of a third cartridge device according to the invention.
FIG. 15 is a schematic drawing of a second embodiment of the probe element.

### DETAILED DESCRIPTION OF PREFERRED EMBODIMENTS

Parts and components having same functions are depicted with same references.

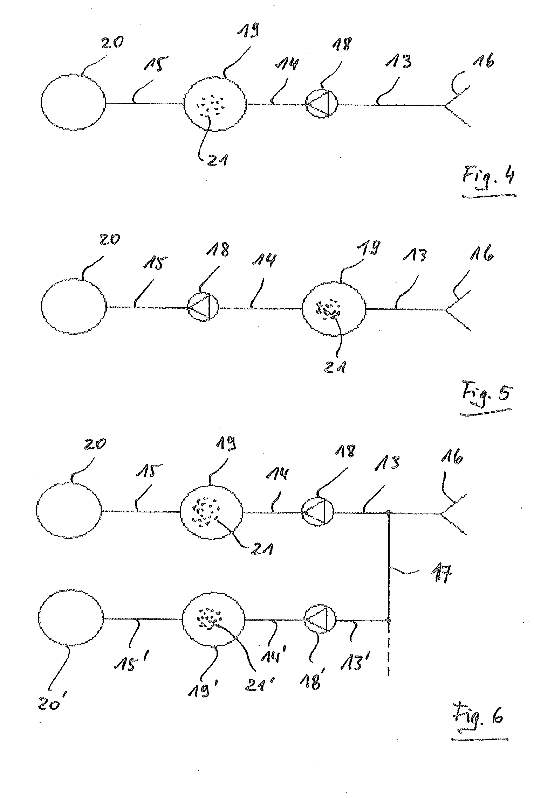

Prior to a detailed description of the preferred embodiments the basic features and a basic practical implementation are summoned as follows. All embodiments refer to a cartridge device 50 (see FIG. 13c) which can be formed in a first embodiment (see FIGs. 4, 5 and 6), in a second embodiment (see FIGs. 7b, 7c and 15) or in a third embodiment (see FIGs. 9 to 10). The cartridge device 50 contains all parts coming into contact with a sample liquid 1 to be tested. These are also reagents the sample liquid, namely blood sample, has to be mixed with for a measurement. The cartridge device 50 is part of a measuring system 40 (see Fig. 13c) to which the cartridge device 50 is attached before measurement. The measuring system 40 also comprises a control apparatus (not shown) which has been adapted to interact with the cartridge device 50 by electrical and/or mechanical means to control flow of sample liquid 1 (see FIG. 7c) and measurements as well as collect data. Furthermore this apparatus contains mechanical and electronic parts required for measurement, data analysis and user interaction. The present invention is not only suitable for thromboelastometry, thromboeleastography and platelet aggregometry but also for other blood tests usually performed regarding surgery.

A first cartridge device 50 will be described with reference to FIGs. 4 and 5. The cartridge device 50 for the measuring system 40 for measuring medical relevant, e.g. viscoelastic, characteristics like coagulation or platelet function of a sample liquid 1, namely a blood sample, comprises a receiving cavity 16 for receiving the sample liquid 1, pump means 18 for pumping the sample liquid, a reagent cavity 19 for storing a reagent 21, a measurement cavity 20 for measuring the sample liquid 1 and a ductwork connecting said cavities. The ductwork comprises an inlet duct 13 from the receiving cavity 16 to the pump means 18, an intermediate duct from the pump means 18 to the reagent cavity 19 and an outlet duct 15 from the reagent cavity 19 to the measurement cavity 20. In a variation said cavities and ducts can be arranged in different ways one of which is shown in FIG. 5, wherein pump means 18 and reagent cavity 19 are changed.

The receiving cavity 16 consists of a cavity within the cartridge device 50. The sample liquid 1 can be applied by means of a syringe, pipette etc, e.g. through a self sealing cap shown as a receiving cavity cover 33a in FIG. 10b. By operating the pump means 18, e.g. by means of the control apparatus mentioned above, the sample liquid is transported to the reagent cavity 19, where the reagent 21 required for measurement is mixed with the sample liquid 1. Further pumping the sample liquid 1 will transfer it into the measurement cavity 20 in which the measurement (described below) is carried out.

In an alternative, the reagent cavity 19 is integral formed with the pump means 18 and/or with the measurement cavity 20 and/or with the ductwork. The transport of the sample liquid 1 can be controlled by said control apparatus.

FIG. 6 shows another variation. Two arrangements of FIG. 4 with only one receiving cavity 16 are arranged in parallel, wherein a first inlet duct 13 communicates with a second inlet duct 13' connected to second pump means 18'. A second intermediate duct 14' leads to a second reagent cavity 19' storing a second reagent 21'. A second outlet duct 15' connects the second reagent cavity 19' to the second measurement cavity 20'. FIG. 6 shows only one possible variation of a plurality of different arrangements easily imagined. The sample liquid 1 is shared among the arrangements in parallel. Controlled by the external control apparatus the shared portions of the sample liquid 1 are mixed with different reagents 21, 21' during transport. It is apparent to a person skilled in the art that in order to achieve a maximum benefit for a user different types of tests can be combined in one cartridge device 50.

In a preferred embodiment the cartridge device 50 comprises four arrangements of FIGs. 4 or 5 having 4 measurement cavities 20, 20'. Thus measurements can be done with different reagents on the same liquid sample or with same reagents as well to check plausibility.

Regarding e.g. blood coagulation there are different reagents available which activate or suppress different parts of the coagulation cascade. Pentapharm GmbH (Munich, Germany) for example amongst others provide tests for intrinsic and extrinsic activation of a blood sample (INTEM or EXTEM respectively), and also a test for extrinsic activation in which the thrombocyte function is suppressed by administration of cytochalasin D (FIBTEM). It is state of the art that it is possible by wise combination of such tests to be able to determine very precisely at which point within the coagulation cascade a problem occurs. This is of great importance in order to determine a proper medication. By comparison of the results on an EXTEM test of a pathologic sample to those of a FIBTEM test of the same sample it is possible to e.g. precisely determine if a coagulation disorder results from lack of fibrinogen or a malfunction of platelets. Generally, there are different typical medical scenarios in which coagulation disorders are very likely to occur. For example coagulation disorders occurring during liver transplantation are merely caused by lack of certain coagulation factors etc., while coagulation disorders during open heart surgery are most likely due to the influence of heparin. This means basically that different medical settings require different coagulation tests. Referring to FIG. 6 it is possible and worthwhile to provide different cartridge devices 50 for different typical operations. It is also possible to combine e.g. an INTEM, an EXTEM and a FIBTEM coagulation test with a platelet aggregometry test within one cartridge. Using such a cartridge the preparation of a measurement which provides almost overall information about the coagulation status of a patient merely requires the two steps of attaching the cartridge device 50 to the measuring system 40 with the external control apparatus and injecting the blood sample as one sample liquid 1. Considering the significance of more complex and time consuming preparation of several thromboelastography or thromboelastometry tests, it is evident that the invention is of great advantage for easier, safer and more accurate POC-tests.

It is important to note that the cartridge devices 50 of the described embodiments are suitable for different diagnostic tests like thromboelastometry, thromboelastography, platelet aggregometry and others. Depending on which type of test or tests the cartridge device 50 is designed for, there are different additional parts required which interact with the sample during measurement and/or an external control apparatus. Possible adaptations for thromboelastometry and platelet aggregometry are described below.

FIG. 7a is a schematic drawing of a first embodiment of a probe element 22 arranged in the measurement cavity 20 (see also FIGs. 10b and 13c). FIGs 7b and 7c show a second cartridge device 50 in form of a cartridge body 30 which comprises only the measurement cavity 20. In the shown example this cavity 20 is accessible via a ductwork 15, 15' through a cavity wall. Alternatively the cavity 20 can be filled through a cover 31, e.g. by injection needles or the like.

The probe element 22 comprises the probe pin 3 (see FIG. 1) which is connected to a flange 24 and a fixing section 25 via an intermediate section 23. The probe element 22 is formed as a rotational part and further comprises a connector section 26 formed as a bore extending within the probe element 22 along its longitudinal axis, which is the rotational axis 5 as well (see FIG. 3).

The probe element 22 is arranged in the measurement cavity 20 of the cartridge body 30 of the cartridge device 50 as shown in FIG. 7b. The measurement cavity 20 is covered by the cover 31 (see also FIGs. 10b and 13c). The cover 31 comprises an opening with fixing means 32 above the measurement cavity 20. The probe element 22 is arranged such that its fixing section 25 corresponding to the fixing means 32 engage with them. In this manner the probe element 22 is detachably fixed to the cover 31. The fixing means 32 in this example are equipped with a circular nose corresponding to a circular notch of the fixing section 25 of the probe element 22. Other fixing means e.g. clip means or the like are possible. The flange 24 is in contact to the inner side of the cover 31.

During attaching the cartridge device 50 to the measuring system 40 (see also FIG. 13c) the shaft 6 of the measuring system 40 (see FIG. 3 and FIGs. 13a...c) is inserted with its bottom portion, an insert section 6a, into the connector section 26. By insertion into the connector section 26 of the probe element 22 the probe element 22 will be detached from the cover 31 not before the insert section 6a is completely inserted in the connector section 26. Then the probe element 22 will be put into in a measuring position as shown in FIG. 7c and kept there. The insert section 6a of the shaft 6 is engaged with the connector section 26 of the probe element 22 e.g. by friction, clip means, thread or the like. In case of a thread the probe element 22 will be hold by the engagement with or perforation of the cover 31. The shaft 6 having a corresponding thread on its insert section 6a will be inserted into the connector section of the probe element 22 by rotation until the insert section 6a will be completely inserted into the connector section 26. Then the shaft 6 can be pushed down and/or rotated together with the fully engaged probe element 22 until the probe element 22 will be detached from the cover 31. FIG. 7c shows the sample liquid 1, which has been pumped into the measurement cavity 20. The probe pin 3 of the probe element 22 is immersed in the sample liquid 1. A measurement as described above can be carried out. After the measurement the cartridge device 50 is detached from the measuring system 40, wherein the shaft 6 is drawn up together with the probe element 22 against the cover 31. The insert section 6a of the shaft 6 will be drawn out of the connector section 26 of the probe element 22 the flange 24 thereof contacting and sealing the opening of the cover 31. Instead of a flange 24 the upper end of the probe element 22 can have a larger diameter than the opening in the cover 31. It is preferred that the insert section 6a of the shaft 6 and the measurement cavity 20, 20' are formed symmetrically.

It is also possible to insert the insert section 6a of the shaft 6 into the connector section 26 of the probe element 22 and push the probe element 22 down until its bottom contacts the bottom of the measurement cavity 20, 20' ensuring that the insert section 6a is completely inserted into the connector section 26. Then the shaft 6 will be moved up into the measuring resp. working position of the probe element 22 as shown in FIG. 7c.

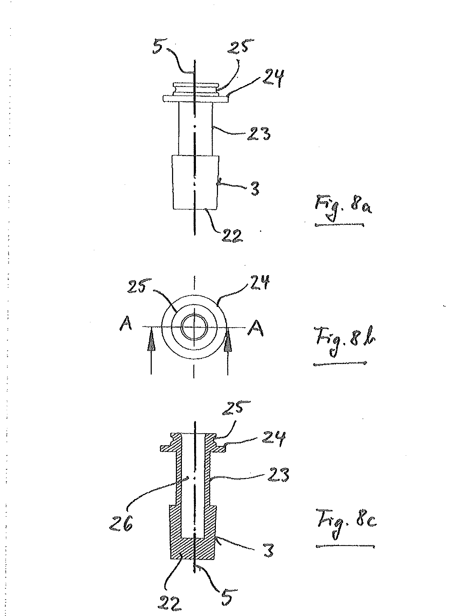

FIGs. 8a...c are technical drawings of a preferred embodiment of the probe element 22 of FIG. 7a. FIG. 8a shows a side view and FIG. 8b shows a top view of the probe element 22 parts of which have been described above regarding FIG. 7a. Finally, FIG. 8c illustrates a sectional view along rotational axis 5. The connector section 26 extends over more than about 75% of the length of the probe element 22.

Now a third cartridge device 50 will be described with reference to FIGs. 9a...d and FIGs. 10a...b.

FIG. 9a is a side view of a second one of a third cartridge device 50. FIG. 9b is a sectional view B-B of the cartridge device 50 of FIG. 9a. FIG. 9c is a sectional view C-C of the cartridge device of FIG. 9a. FIG. 9b is a sectional view D-D of the cartridge device of FIG. 9a. FIG. 10a is a top view of the cartridge device of FIG. 9a. FIG. 10b is a sectional view E-E of the cartridge device of FIG. 10a.

The cartridge device 50 of this example is equipped with the ductwork 13 and 15. The ducts are formed with a diameter of approximately 1mm. The ductwork requires that the cartridge device 50 comprises two parts: the cartridge body 30 and the cover 31, which are glued or welded together to obtain a leak-proof device. The cartridge body 30 is relative rigid and the cover 31 is formed as an elastic part. So it is possible to integrate the pump means 18 into the cover 31. Moreover, the cover 31 covers the receiving cavity 16 with the receiving cavity cover 33a and forms a type of liner wall 33 and a separation wall 34 forming an inlet for the inlet duct 13 within the receiving cavity 16. The receiving cavity cover 33a might act as a self seal for injection of a sample liquid 1 by a syringe for example. The cover 31 forms top parts of the ductwork 13 and 15 and a cover of the measurement cavity 20 (see also FIGs. 7b...c). In this example the pump means 18 comprises a pump membrane 35 formed by the cover 31. The pump membrane 35 cooperates with a pump cavity 36 formed with a pump cavity bottom 36a in the cartridge body 30 below the pump membrane 35.

A reagent cavity 19, 19' is formed, e.g. by sections of the ductwork or/and the pump means 18, 18' in which the reagents can be stored resp. deposited, especially on the pump cavity bottom 36a, for example.

The pump means 18 will now be described with reference to FIGs. 11a...b and FIG.12.

FIG. 11a is a sectional view of the pump means 18, 18' of the cartridge device 50, FIG. 11b is a sectional view of the pump means 18 of FIG. 11a in operated position, and FIG. 12 is a schematic top view of the pump means 18 of FIG. 11a.

In this example the pump cavity 36 is connected to the inlet duct 13 via an inlet valve 37 and to the outlet valve via an outlet valve 38. Actuation of the pump membrane 35 (shown in FIG. 11b in a working cycle) by an appropriate actuating means (not shown) of the control apparatus the pump means 18 will create a directed flow of the sample liquid 1 in a flow direction 39 depicted by the arrows. The pump membrane 35 being an integrated part of the cover 31 can be made of the cover material or a part made of another material integrally manufactured with the cover 31, e.g. two components manufacturing. The valves 37, 38 can be a type of non-return valve. FIG. 12 shows a top view of the pump means in a schematic way.

An external force exerted on the pump membrane 35 increase the pressure within the pump cavity 36 and opens outlet valve 38 and closes inlet valve 37. Releasing the external force the elastic pump membrane 35 returns into the position shown in FIG. 11a whereby outlet valve 38 will be closed and inlet valve 37 opened to let sample liquid 1 into the pump cavity 36. This mechanism is state of the art according to DE10135569. In context with the present invention the actuation means of the control apparatus activating the pump membrane 35 from outside has the advantage of strict separation between those parts coming into contact with the sample liquid 1 and the control apparatus. At the same time the total number of parts required for the cartridge device 50 being a disposable part as well is kept on a minimum.

Now the measuring system 40 according to the invention is described in an embodiment with reference to FIGs. 13a...c.

FIG. 13a is a side view of an embodiment of the measuring system 40, FIG. 13b is a top view of the measuring system 40 of FIG. 13a, and FIG. 13c is a sectional view H-H of the measuring system 40 of FIG. 13b.

The measuring system 40 comprises an interface element 41 to which the cartridge device 50 is attached and fixed. The interface element 41 is shown in FIG. 13a to 13a in way of example as a base plate. The function of the interface element 41 is to support the shaft 6 and to maintain its position and thus the position of the probe element 22 fixed to the insert section 6a in a measurement position. The interface element 41 can be connected to the whole cover 31 as shown in FIG. 13a to 13c or only to parts of the cover 31, e.g. surrounding the rotation axis 5. The shaft 6 is rotatable supported in a bearing 7 within a shaft passage 44 (FIG. 13c) and can be rotated around the rotation axis 5 (see also FIG. 3) by driving the spring 9 via driving means (not shown). The detecting means 10 cooperate with the mirror 8 fixed on the shaft 3, also shown in FIG. 3. The control apparatus mentioned above is not shown as well, but easy to imagine. Its actuation and/or operating means can access the pump means 18 through an opening pump access 42 in the interface element 41. The receiving cavity 16 is accessible through another inlet opening 43. These and other different passages or passage ways of the interface element 41 to have access to the cartridge device 50 and/or its cover 31 are illustrated by FIG. 13b as a top view of the measuring system 40 of FIG. 13a. Passage holes 44a are arranged next to the rotational axis 5 to form an access to the cover 31 above the measurement cavity 20, 20', e.g. for injection of liquid sample or reagents. Additional access passage holes can be arranged in the interface element 41, e.g. above the ductwork to access said ductwork.

FIG. 13c illustrates a sectional view H-H of FIG. 13b showing the mounted cartridge device 50 and the measuring system 40. The shaft 6 with its insert section 6a is inserted into the probe element 22 and keeps it in a measurement position as mentioned above. It comprises only one measurement cavity 20, but it is apparent to a person skilled in the art that modifications and combinations can be carried out in different ways.

Thus it is possible to e.g. arrange a reagent receptacle 19b in a blister receptacle e.g. as shown in FIG. 14 which is a sectional view of the reagent receptacle 19b of a third embodiment of the cartridge device 50 according to the invention. The receptacle 19b contains reagent 21 hold within a chamber defined by a blister cover 49, a bottom part 48 and a frame 47 hold in a retaining ring 46 within an reagent cover opening 45 in the cover 31 above the reagent cavity 19, 19' with a reagent cavity bottom 19a, 19a'. Upon exertion of a force by the control apparatus onto the blister cover 49 the bottom part 48 will open and discharge the reagent 21 into the reagent cavity 19, 19'. The receptacle 19b can be fixed to the cover by e.g. clip means as depicted. The frame 47 can be a reinforced ring. The blister cover 49 is reinforced so that it will not break when a force is exerted on it. Thus the leak-tightness of the cartridge device 50 will be ensured. In this way a unitized construction system can be made, wherein the respective reagents can be easily integrated into the cartridge device 50. It is also advantageous that the reagents can be designed as a small component being cooled resp. transported and supplied easily.

It is also possible to insert reagent receptacles into provided cavities being connected to the ductwork. The reagents can be designed as globules with an appropriate diameter so that they cannot flow through openings into the ductwork before being dissolved by the sample liquid.

FIG. 15 is a schematic drawing of a second embodiment of a probe element 22'. The probe element 22' is arranged in the measurement cavity 20. The probe pin 3 is provided with a dimple 29 at its bottom side. The dimple 29 forms with a nose 29a a toe bearing to support the probe element 22'. The probe element 22' is similar to the probe element 22 of FIG. 7a, but has no fixing section 25, only the flange 24. The connector section 26 comprises a top end formed with an insertion guide 27 for the insertion section 6a of the shaft. The probe element 22' is hold in the measurement cavity 20 in a specific manner so that the insertion section 6a of the shaft 6 can be inserted easily through an opening 32a of the cover 31 which has no fixing means. The insertion section 6a can engage with a groove 28 inside the connector section 26 of the probe element 22'. After that engagement which is supported by the toe bearing the shaft 6 will be drawn up together with the probe element 22' in the measuring position. It is a matter of fact that other engagement means can be used.

### References

- 1: Sample liquid
- 2: Cup
- 3: Probe pin
- 4: Torsion wire
- 5: Rotation axis
- 6: Shaft
- 6a: Insert section
- 7: Bearing
- 8: Mirror
- 9: Spring
- 10: Detecting means
- 11: Base plate
- 12: Cup holder
- 13, 13': Inlet duct
- 14, 14': Intermediate duct
- 15, 15': Outlet duct
- 16, 16': Receiving cavity
- 17: Branch duct
- 18, 18': Pump means
- 19, 19': Reagent cavity
- 19a, 19'a: Reagent cavity bottom
- 19b: Reagent receptacle
- 20, 20': Measurement cavity
- 21, 21': Reagent
- 22, 22': Probe element
- 23: Intermediate section
- 24: Flange
- 25: Fixing section
- 26: Connector section
- 27: Insertion guide
- 28: Groove
- 29: Dimple
- 29a: Nose
- 30: Cartridge body
- 31: Cover
- 32: Fixing means
- 32a: Opening
- 33: Wall
- 33a: Receiving cavity cover
- 34: Separation wall
- 35: Pump membrane
- 36: Pump cavity
- 36a: Pump cavity bottom
- 37: Inlet valve
- 38: Outlet valve
- 39: Flow direction
- 40: Measuring system
- 41: Interface element
- 42: Pump access
- 43: Inlet opening
- 44: Shaft passage
- 44a: Passage hole
- 45: Reagent cover opening
- 46: Retaining ring
- 47: Frame
- 48: Bottom foil
- 49: Blister cover
- 50: Cartridge device

## Claims

1. A system for measuring viscoelastic characteristics of a blood sample (1), the system comprising:
a cartridge device (50) which comprises:
a plurality of receiving cavities (16, 16') for receiving blood sample (1);
a plurality of reagent cavities (19, 19') each configured to store a reagent (21, 21') to be mixed with blood sample (1) for measurement; and
a plurality of measurement cavities (20, 20'), wherein there is one probe for each measurement cavity (20, 20');
at least one pump means (18, 18') for transporting blood sample (1);
ductwork (13, 13'; 14, 14'; 15, 15'; 17) for connecting the plurality of receiving cavities (16, 16'), the plurality of reagent cavities (19, 19'), the at least one pump means (18, 18'), and the plurality of measurement cavities (20, 20'), wherein the ductwork (13, 13'; 14, 14'; 15, 15'; 17) comprises inlet ducts (13, 13') connecting the receiving cavities (16, 16') to the at least one pump means (18, 18'), ducts (14, 14', 15, 15') connecting the at least one pump means (18, 18') to the reagent cavities (19, 19') and connecting the reagent cavities (19, 19') to the measurement cavities (20, 20'), wherein by operating the at least one pump means (18, 18'), blood sample (1) is transported to the reagent cavities (19, 19'), where the reagent (21, 21') is to be mixed with blood sample (1); and
a cover (31), wherein the cover (31) covers and at least partially forms said cavities (16, 16'; 19, 19', 20, 20') and said ductwork (13, 13'; 14, 14'; 15, 15'; 17) and forms at least partially the at least one pump means (18, 18');
wherein the reagent (21, 21') to be stored in each of the plurality of reagent cavities (19, 19') comprises a reagent or combination of reagents, the plurality of reagent cavities (19, 19') including at least:
a first cavity comprising a reagent to activate coagulation; and
a second cavity comprising a reagent to activate coagulation and one or more reagents configured for suppressing thrombocyte function; and
wherein a respective probe is configured to measure a viscoelastic characteristic of a mixture of blood sample and reagent in a respective measurement cavity (20, 20').

2. The system of claim 1, wherein the reagent in the first cavity and the reagent in the second cavity activate different parts of a coagulation cascade.

3. The system of claim 2, wherein the plurality of reagent cavities (19, 19') comprises a third cavity, wherein the third cavity comprises a reagent that is different from reagents in the first cavity and the second cavity.

4. The system of claim 3, wherein a reagent in one of the first cavity and the second cavity is for extrinsic activation of a portion of the blood sample; a reagent in the third cavity is for intrinsic activation of a portion of the blood sample; and the one or more reagents are for suppressing thrombocyte function include cytochalasin D.

5. The system of any one of the claims 1 to 4, wherein the system includes control apparatus configured for interacting with the cartridge device (50) to move portions of the blood sample through the cartridge device (50).

6. The system of any one of the claims 1 to 5, wherein the probe is configured to perform a viscoelastic method of testing to measure changing viscoelastic properties of the mixture of a portion of the blood sample and reagent in the measurement cavity (20, 20').

7. The system of any one of the claims 1 to 6, wherein the cartridge device (50) is configured for testing that includes thromboelastometry, thromboelastography, or platelet aggregometry.

8. The system of any one of the claims 1 to 7, wherein the cartridge is configured to receive the blood sample (1) via a needle.

9. The system of any one of the claims 1 to 8, wherein the plurality of reagent cavities (19, 19') comprises four reagent cavities (19, 19').

10. The system of any one of the claims 1 to 9, wherein the cartridge comprises four measurement cavities (20, 20').

11. The system of any one of the claims 1 to 10, wherein reagent or a combination of reagents for each of the reagent cavities (19, 19') is stored in the cartridge device (50) in solid form prior to interacting with the blood sample.

## Patentansprüche

1. System zum Messen der viskoelastischen Eigenschaften einer Blutprobe (1), wobei das System Folgendes umfasst:
eine Kartuschenvorrichtung (50), aufweisend:
eine Vielzahl von Aufnahmekavitäten (16, 16') zum Aufnehmen der Blutprobe (1);
eine Vielzahl von Reagenzkavitäten (19, 19'), die jeweils so konfiguriert sind, dass sie ein Reagenz (21, 21') speichern, das mit der Blutprobe (1) zur Messung gemischt werden soll; und
eine Vielzahl von Messkavitäten (20, 20'), wobei für jede Messkavität (20, 20') eine Sonde vorhanden ist;
mindestens eine Pumpeinrichtung (18, 18') zum Transportieren der Blutprobe (1);
ein Kanalsystem (13, 13'; 14, 14'; 15, 15'; 17) zum Verbinden der Vielzahl von Aufnahmekavitäten (16, 16'), der Vielzahl von Reagenzkavitäten (19, 19'), der mindestens einen Pumpeinrichtung (18, 18') und der Vielzahl von Messkavitäten (20, 20'), wobei das Kanalsystem (13, 13'; 14, 14'; 15, 15'; 17) Einlasskanäle (13, 13') umfasst, die die Aufnahmekavitäten (16, 16') mit der mindestens einen Pumpeinrichtung (18, 18') verbinden, Kanäle (14, 14', 15, 15'), die die mindestens eine Pumpeinrichtung (18, 18') mit den Reagenzkavitäten (19, 19') verbinden und die Reagenzkavitäten (19,19') mit den Messkavitäten (20, 20') verbinden, wobei durch Betätigen der mindestens einen Pumpeinrichtung (18, 18') die Blutprobe (1) zu den Reagenzkavitäten (19, 19') transportiert wird, wo das Reagenz (21, 21') mit der Blutprobe (1) gemischt werden soll; und
eine Abdeckung (31), wobei die Abdeckung (31) die Kavitäten (16, 16'; 19, 19', 20, 20') und das Kanalsystem (13, 13'; 14, 14'; 15, 15'; 17) abdeckt und mindestens teilweise ausbildet und zumindest teilweise die mindestens eine Pumpeinrichtung (18, 18') ausbildet;
wobei das Reagenz (21, 21'), das in jedem der Vielzahl von Reagenzkavitäten (19, 19') gelagert werden soll, ein Reagenz oder eine Kombination von Reagenzien umfasst, wobei die Vielzahl von Reagenzkavitäten (19, 19') beinhalten:
eine erste Kavität, die ein Reagenz zum Aktivieren der Gerinnung beinhaltet; und/oder
eine zweite Kavität, die ein Reagenz zum Aktivieren der Gerinnung und ein oder mehrere Reagenzien, die zum Unterdrücken der Thrombozytenfunktion konfiguriert sind, beinhaltet; und
wobei eine jeweilige Sonde so konfiguriert ist, dass sie eine viskoelastische Eigenschaft eines Gemischs aus Blutprobe und Reagenz in einer jeweiligen Messkavität (20, 20') misst.

2. System nach Anspruch 1, wobei das Reagenz in der ersten Kavität und das Reagenz in der zweiten Kavität unterschiedliche Teile einer Gerinnungskaskade aktivieren.

3. System nach Anspruch 2, wobei die Vielzahl von Reagenzkavitäten (19, 19') eine dritte Kavität umfasst, wobei die dritte Kavität ein Reagenz beinhaltet, das sich von den Reagenzien in der ersten Kavität und in der zweiten Kavität unterscheidet.

4. System nach Anspruch 3, wobei ein Reagenz in der ersten und/oder zweiten Kavität zur extrinsischen Aktivierung eines Abschnitts der Blutprobe dient; ein Reagenz in dem dritten Hohlraum zur intrinsischen Aktivierung eines Abschnitts der Blutprobe dient; und das eine oder mehrere Reagenzien zum Unterdrücken der Thrombozytenfunktion Cytochalasin D beinhalten.

5. System nach einem der Ansprüche 1 bis 4, wobei das System eine Steuervorrichtung beinhaltet, die so konfiguriert ist, dass sie mit der Kartuschenvorrichtung (50) interagiert, um Abschnitte der Blutprobe durch die Kartuschenvorrichtung (50) zu bewegen.

6. System nach einem der Ansprüche 1 bis 5, wobei die Sonde so konfiguriert ist, dass sie ein viskoelastisches Testverfahren durchführt, um die sich ändernden viskoelastischen Eigenschaften des Gemischs aus einem Abschnitt der Blutprobe und dem Reagenz in der Messkavität (20, 20') zu messen.

7. System nach einem der Ansprüche 1 bis 6, wobei die Kartuschenvorrichtung (50) für Tests konfiguriert ist, die Thromboelastometrie, Thromboelastographie oder Thrombozytenaggregometrie beinhalten.

8. System nach einem der Ansprüche 1 bis 7, wobei die Kartusche so konfiguriert ist, dass sie die Blutprobe (1) über eine Nadel aufnimmt.

9. System nach einem der Ansprüche 1 bis 8, wobei die Vielzahl von Reagenzkavitäten (19, 19') vier Reagenzkavitäten (19, 19') umfasst.

10. System nach einem der Ansprüche 1 bis 9, wobei die Kartusche vier Messkavitäten (20, 20') aufweist.

11. System nach einem der Ansprüche 1 bis 10, wobei das Reagenz oder eine Kombination von Reagenzien für jeden der Reagenzkavitäten (19, 19') in der Kartuschenvorrichtung (50) in fester Form gelagert wird, bevor es mit der Blutprobe interagiert.

## Revendications

1. Système permettant de mesurer des caractéristiques viscoélastiques d'un échantillon de sang (1), le système comprenant :
un dispositif à cartouche (50) comprenant :
une pluralité de cavités de réception (16, 16') destinées à recevoir l'échantillon de sang (1) ;
une pluralité de cavités (19, 19') de réactif conçues chacune pour stocker un réactif (21, 21') à mélanger avec l'échantillon de sang (1) à des fins de mesure ; et
une pluralité de cavités de mesure (20, 20'), chaque cavité de mesure (20, 20') étant dotée d'une sonde ;
au moins un moyen de pompage (18, 18') destiné à transporter l'échantillon de sang (1) ;
un réseau de conduits (13, 13'; 14, 14'; 15, 15'; 17) destiné à relier la pluralité de cavités de réception (16, 16'), la pluralité de cavités (19, 19') de réactif, le ou les moyens de pompage (18, 18') et la pluralité de cavités de mesure (20, 20'), le réseau de conduits (13, 13'; 14, 14'; 15, 15'; 17) comprenant des conduits d'entrée (13, 13') reliant les cavités de réception (16, 16') au ou aux moyens de pompage (18, 18'), des conduits (14, 14'; 15, 15') reliant le ou les moyens de pompage (18, 18') aux cavités (19, 19') de réactif et reliant les cavités (19, 19') de réactif aux cavités de mesure (20, 20'), et, au moyen de l'actionnement du ou des moyens de pompage (18, 18'), l'échantillon de sang (1) étant transporté aux cavités (19, 19') de réactif, dans lesquelles le réactif (21, 21') est amené à se mélanger avec l'échantillon de sang (1) ; et
un couvercle (31), le couvercle (31) recouvrant et formant, au moins partiellement, lesdites cavités (16, 16'; 19, 19'; 20, 20') et ledit réseau de conduits (13, 13'; 14, 14'; 15, 15'; 17), et formant, au moins partiellement, le ou les moyens de pompage (18, 18') ;
dans lequel le réactif (21, 21'), à être stocké dans chaque cavité parmi la pluralité de cavités (19, 19') de réactif, comprend un réactif ou une combinaison de réactifs, la pluralité de cavités (19, 19') de réactif comportant au moins :
une première cavité comprenant un réactif destiné à activer la coagulation ; et
une deuxième cavité comprenant un réactif destiné à activer la coagulation et un ou plusieurs réactifs conçus pour supprimer la fonction thrombotique ; et
dans lequel une sonde respective est conçue pour mesurer une caractéristique viscoélastique d'un mélange d'échantillon de sang et de réactif dans une cavité de mesure (20, 20') respective.

2. Le système de la revendication 1, dans lequel le réactif dans la première cavité et le réactif dans la deuxième cavité activent différentes parties d'une cascade de coagulation.

3. Le système de la revendication 2, dans lequel la pluralité de cavités (19, 19') de réactif comprend une troisième cavité, la troisième cavité comprenant un réactif différent des réactifs dans la première cavité et dans la deuxième cavité.

4. Le système de la revendication 3, dans lequel un réactif dans une cavité, parmi la première cavité et la deuxième cavité, a pour but l'activation extrinsèque d'une portion de l'échantillon de sang ; un réactif dans la troisième cavité a pour but l'activation intrinsèque d'une portion de l'échantillon de sang ; et le ou les réactifs ayant pour but la suppression de la fonction thrombotique comprennent de la cytochalasine D.

5. Le système de l'une quelconque des revendications 1 à 4, dans lequel le système comporte un appareil de commande conçu pour interagir avec le dispositif à cartouche (50) afin de déplacer des portions de l'échantillon de sang à travers le dispositif à cartouche (50).

6. Le système de l'une quelconque des revendications 1 à 5, dans lequel la sonde est conçue pour effectuer un procédé d'essai viscoélastique afin de mesurer des propriétés viscoélastiques changeantes du mélange d'une portion de l'échantillon de sang et de réactif dans la cavité de mesure (20, 20').

7. Le système de l'une quelconque des revendications 1 à 6, dans lequel le dispositif à cartouche (50) est conçu pour effectuer des essais comprenant une thromboélastométrie, une thromboélastographie ou une agglutinométrie de plaquettes.

8. Le système de l'une quelconque des revendications 1 à 7, dans lequel la cartouche est conçue pour recevoir l'échantillon de sang (1) par l'intermédiaire d'une aiguille.

9. Le système de l'une quelconque des revendications 1 à 8, dans lequel la pluralité de cavités (19, 19') de réactif comprend quatre cavités (19, 19') de réactif.

10. Le système de l'une quelconque des revendications 1 à 9, dans lequel la cartouche comprend quatre cavités de mesure (20, 20').

11. Le système de l'une quelconque des revendications 1 à 10, dans lequel le réactif ou une combinaison de réactifs, pour chaque cavité (19, 19') de réactif, sont stockés dans le dispositif à cartouche (50) sous forme solide avant d'interagir avec l'échantillon de sang.
